(19) Europäisches Patentamt
European Patent Office
Office européen des brevets

(11) Publication number: 0 321 364 A2

(12) EUROPEAN PATENT APPLICATION

(21) Application number: 88420413.2

(51) Int. Cl.⁴: C 07 D 501/20

(22) Date of filing: 07.12.88

(30) Priority: 12.12.87 JP 313169/87

(43) Date of publication of application: 21.06.89 Bulletin 89/25

(84) Designated Contracting States: AT BE CH DE ES FR GB IT LI LU NL SE

(71) Applicant: ZAIDAN HOJIN BISEIBUTSU KAGAKU KENKYU KAI
14-23, Kamiosaki 3-chome
Shinagawa-ku Tokyo (JP)

(72) Inventor: Takeuchi, Tomio
1-11 Higashi Gotanda 5-Chome
Shinagawa-ku Tokyo (JP)

Kondo, Shinichi
1157-1-801 Ichigao-cho Midori-ku
Yokohama-shi Kanagawa-ken (JP)

Ikeda, Daishiro
29-8-107 Yoyogi 5-Chome
Shibuya-ku Tokyo (JP)

Ko, Sehei
17 Denenchofu Honcho 3-Chome
Ohta-ku Tokyo (JP)

Koyama, Yoshiyuki
9-20-1-D Sanno 3-Chome
Ohta-ku Tokyo (JP)

(74) Representative: Maureau, Bernard et al
Cabinet GERMAIN & MAUREAU 20, boulevard E. Deruelle
B.P. 3011
F-69392 Lyon Cédex 03 (FR)

# (54) Orally administrable cephalosporin derivatives, and the production and uses thereof.

(57) As new antibacterial compounds are provided a cephalosporin derivative having the general formula (I)

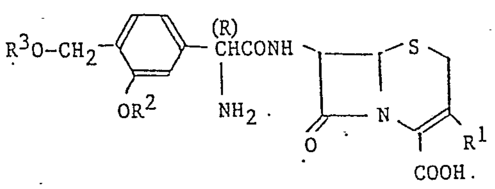

(I)

wherein $R^1$ is a lower alkyl group, a lower alkoxymethyl group or a lower alkenyl group, and $R^2$ and $R^3$ are each a hydrogen atom or a lower alkyl group, and pharmaceutically acceptable salt thereof.

This new cephalosporin derivative of the formula (I) is useful as an orally administrable antibacterial agent which exhibits an excellent antibacterial activity, high absorbability through the digestive tracts of animal and high urinary recovery thereof.

EP 0 321 364 A2

**Description**

**Orally administrable cephalosporin derivatives and the production and uses thereof**

SUMMARY OF THE INVENTION

This invention relates to new cephalosporin derivatives, and more particularly, to such orally administrable new cephalosporin compounds which have improved antibacterial activity and exhibit high absorption by digestive tracts of an animal, including human, when orally administered, and which are useful as a therapeutic antibacterial agent. This invention also relates to processes for the production of these new cephalosporin derivatives. This invention further includes a pharmaceutical composition containing the new cephalosporin derivatives as active ingredient.

BACKGROUND OF THE INVENTION

Many cephalosporin antibiotics are known to be highly active against a variety of gram-positive and gram-negative bacteria, and are useful for the therapeutic treatment of various bacterial infections. Some of these known cephalosporin antibiotics are absorbable through the digestive tracts of an animal and hence orally administrable. The examples of such orally administrable cephalosporin compounds include Cefalexin, Cefaclor and Cefadroxil, and most recently there is reported BMY-28100 substance {"Journal of Antibiotics", Vol. 40,991 (1987)}. These orally administrable cephalosporin compounds commonly have such a chemical structure that the amino group at the 7-position of their cephem nucleus is acylated with D-phenylglycine or D-(p-hydroxyphenyl)glycine. These known orally administrable cephalosporin compounds still do not exhibit an entirely satisfactorily high antibacterial activity against some strains of bacteria which have been a target of clinical treatments of bacterial infections. It is therefore always demanded that new cephalosporin compounds having any further enhanced antibacterial activity are developed and provided for uses in therapeutic treatment of bacterial infections.

We, the present inventors, already provided such novel cephalosporin derivatives that the amino group at the 7-position of their cephem nucleus has been acylated with an immuno-modifier compound, forphenicinol, which is obtained from the reduction of a natural occuring known compound, forphenicine {"Journal of Antibiotic", Vol. 35, 1042, (1982)}. These cephalosporin derivatives, however, do not exhibit a satisfactorily high antibacterial activity against a variety of bacteria.

Then, we have made extensively our researches in an attempt to provide orally administrable another cephalosporin derivatives by chemical synthesis, and we have tried to use an optical isomer of the forphenicinol, namely D-forphenicinol, that is, (R)-2-amino-2-(3-hydroxy-4-hydroxymethylphenyl)acetic acid {"Journal of Antibiotics", Vol. 35, 1500, (1982)} as the acylating agent for the 7-amino group of the cephem nucleus. As a result, we have succeeded in synthesizing new cephalosporin compounds having the general formula (I) as shown below, and we have also found that these new cephalosporin compounds now synthesized exhibit excellent antibacterial activity and high absorbability in vivo, as well as a high rate of their recovery in urine. We have thus accomplished this invention.

DETAILED DESCRIPTION OF THE INVENTION

In a first aspect of this invention, therefore, there is provided a new cephalosporin derivative having the general formula (I)

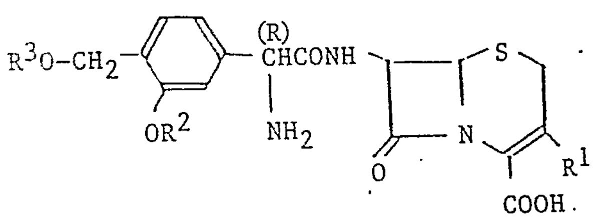

(I)

wherein $R^1$ is a lower alkyl group, a lower alkoxymethyl group or a lower alkenyl group, and $R^2$ and $R^3$ are each a hydrogen atom or a lower alkyl group, and pharmaceutically acceptable salt thereof.

The term "lower alkyl group" as used herein for the group $R^1$, $R^2$ or $R^3$ in the general formula (I) means an alkyl group having 1 to 6 carbon atoms, such as methyl, ethyl, n-propyl, isopropyl, n-butyl, isobutyl, tert-butyl, n-pentyl and the like. The term "lower alkoxymethyl group" means a methyl group substituted with a lower ($C_1$ - $C_6$) alkoxy group, such as methoxymethyl, ethoxymethyl, n-propoxymethyl, isopropoxymethyl, n-butoxymethyl, isobutoxymethyl and the like. The term "lower alkenyl group" means an alkenyl group having 2 to 6 carbon atoms, such as vinyl, cis-n-propenyl, trans-n-propenyl, cis-n-butenyl, trans-n-butenyl and the like.

The 4-carboxyl group of the cephalosporin compound of the formula (I) may form a pharmaceutically acceptable salt (the carboxylate) by reaction with a pharmaceutically acceptable cation, such as an alkali metal cation, especially sodium and potassium cation, as well as an alkaline earth metal cation, such as calcium and magnesium cation. The 4-carboxyl group of the cephalosporin compound of the formula (I) may also form an

ester (the carboxylate) with a pharmaceutically acceptable ester-forming group, which includes a lower alkanoyloxyalkyl group such as acetoxymethyl, 1-acetoxyethyl and pivaloyloxymethyl, as well as a lower alkoxycarbonyloxyalkyl group such as 1-(ethoxycarbonyloxy)ethyl, and phthalidyl group and (5-methyl-2-oxo-1,3-dioxolen-4-yl)methyl group, and which may preferably be an ester-forming group readily and hydrolytically cleavable in vivo in the body of animal after the oral administration of the ester compound.

Examples of the new cephalosporin compounds of the formula (I) according to this invention are as follows;

(1) 7-{(R)-2-amino-2-(3-hydroxy-4-hydroxymethylphenyl) acetamido}-3-methyl-3-cephem-4-carboxylic acid, namely the compound represented by the general formula (I) where $R^1$ is a methyl group and $R^2$ and $R^3$ are each a hydrogen atom (the compound as produced in Example 1 given hereinafter), and a pharmaceutically acceptable salt thereof.

(2) 7-{(R)-2-amino-2-(3-hydroxy-4-hydroxymethylphenyl) acetamido}-3-methoxymethyl-3-cephem-4-carboxylic acid, namely the compound represented by the general formula (I) where $R^1$ is a methoxymethyl group and $R^2$ and $R^3$ are each a hydrogen atom (the compound as produced in Example 2 given hereinafter), and a pharmaceutically acceptable salt thereof.

(3) 7- {(R)-2-amino-2-(3-hydroxy-4-hydroxymethylphenyl) acetamido}-3-vinyl-3-cephem-4-carboxylic acid, namely the compound represented by the general formula (I) where $R^1$ is a vinyl group of the formula-$CH=CH_2$ and $R^2$ and $R^3$ are each a hydrogen atom (the compound as produced in Example 3 given hereinafter), and a pharmaceutically acceptable salt thereof.

(4) 7- {(R)-2-amino-2-(3-hydroxy-4-hydroxymethylphenyl) acetamido)-3- {(Z)-1-propenyl)-3-cephem-4-carboxylic acid, namely the compound represented by the general formula (I) where $R^1$ is a propenyl group (cis-form) of the formula-$CH=CHCH_3$ and $R^2$ and $R^3$ are each a hydrogen atom (the compound as produced in Example 4 given hereinafter), and a pharmaceutically acceptable salt thereof.

(5) 7-{(R)-2-amino-2-(4-hydroxymethyl-3-methoxyphenyl) acetamido}-3- {(Z)-1-propenyl}-3-cephem-4-carboxylic acid, namely the compound represented by the general formula (I) where $R^1$ is a propenyl group (cis-form) of the formula -$CH=CHCH_3$, $R^2$ is a methyl group and $R^3$ is a hydrogen atom (the compound as obtained in Example 5 given hereinafter), and a pharmaceutically acceptable salt thereof, and

(6) 7-{(R)-2-amino-2-(3-methoxy-4-methoxymethylphenyl)acetamido}-3-{(Z)-1-propenyl}-3-cephem-4-carboxylic acid, namely the compound represented by the general formula (I) where $R^1$ is a propenyl group (cis-form) of the formula-$CH=CHCH_3$ and $R^2$ and-$R^3$ are each a methyl group (the compound obtained in Example 6 given hereinafter), and a pharmaceutically acceptable salt thereof.

These new cephalosporin compounds according to this invention and having the general formula (I) are all in the form of colorless powder or crystalline powder substance which is soluble in water, methanol and ethanol.

The antibacterial activities of the cephalosporin compounds of the formula (I) according to this invention, specifically the final product compounds of the Examples 1 to 6 given hereinafter, were assayed in term of their minimum inhibitory concentrations (MIC; mcg/ml) against various test bacteria, as determined according to a standard serial dilution method in Müller-Hinton agar medium (a product of Difico company), where the incubation was made at 37°C for 18 hours. For the comparison purpose, the antibacterial spectrum of a known compound, Cefalexin (abbreviated as CEX) was also assayed in the same way as above. The results obtained are shown in Table 1 below.

Table 1

| Test Organisms | MIC (mcg/ml) | | | | | | |
|---|---|---|---|---|---|---|---|
| | Example 1 Compound | Example 2 Compound | Example 3 Compound | Example 4 Compound | Example 5 Compound | Example 6 Compound | CEX (Comparative) |
| Staphylococcus aureus FDA209P JC-1 | 3.13 | 1.56 | 1.56 | 1.56 | 1.56 | 0.78 | 3.13 |
| " Terajima | 0.2 | 0.2 | 0.2 | 0.1 | 0.1 | 0.1 | 0.2 |
| " MS353 | 3.13 | 1.56 | 1.56 | 1.56 | 0.78 | 1.56 | 3.13 |
| Micrococcus luteus ATCC9341 | 0.1 | 0.1 | 0.2 | 0.1 | 0.025 | 0.025 | 0.1 |
| Bacillus subtilis ATCC6633 | 0.78 | 0.2 | 0.05 | 0.2 | 0.05 | 0.05 | 0.78 |
| Pseudomonas aeruginosa IFO3445 | >100 | >100 | >100 | >100 | >100 | >100 | >100 |
| Escherichia coli NIHJ-JS-2 | 12.5 | 12.5 | 6.25 | 6.25 | 6.25 | 25 | 6.25 |
| " K-12 C600 | 12.5 | 12.5 | 6.25 | 3.13 | 3.13 | 6.25 | 6.25 |
| Klebsiella pneumoniae PCI602 | 6.25 | 6.25 | 3.13 | 0.78 | 0.78 | 1.56 | 3.13 |
| Salmonella typhimurium IID971 | 6.25 | 6.25 | 3.13 | 1.56 | 3.13 | 12.5 | 6.25 |
| " typhi 901 | 6.25 | 6.25 | 3.13 | 0.78 | 1.56 | 12.5 | 3.13 |
| " paratyphi 1015 | 12.5 | 12.5 | 3.13 | 1.56 | 0.78 | 3.13 | 6.25 |
| " schottmulleri 8006 | 12.5 | 6.25 | 3.13 | 1.56 | 0.39 | 1.56 | 6.25 |
| " enteritidis G14 | 50 | 6.25 | 3.13 | 0.78 | 0.78 | 6.25 | >100 |
| Proteus morganii IFO3848 | >100 | 50 | 50 | 1.56 | 3.13 | 1.56 | 25 |
| " rettgeri IFO3850 | 12.5 | 25 | 50 | 1.56 | 3.13 | 12.5 | 12.5 |
| " vulgaris OX-19 | 100 | 100 | 25 | 25 | 50 | 100 | 12.5 |
| Rms212/Escherichia coli K-12 W3630 * | 12.5 | 50 | 12.5 | 12.5 | 12.5 | 100 | 12.5 |
| Rms823/ " * | 100 | >100 | >100 | 100 | >100 | >100 | 12.5 |
| Rms213/ " * | 12.5 | 12.5 | 12.5 | 6.25 | 25? | 12.5 | 12.5 |
| Rte16/ " * | 12.5 | 25 | 12.5 | 12.5 | 3.13 | 25 | 12.5 |
| Rms139/MI Pseudomonas aeruginosa * | >100 | >100 | >100 | >100 | 100 | >100 | >100 |
| Klebsiella oxytosa GN10560 ** | 100 | >100 | >100 | >100 | >100 | >100 | >100 |

* penicillinase producing strain
** cefuroximase (hydrolysed cephalosporin having methoxyiminoacetyl side chain at 7-position) producing strain

Generally, when such a cephalosporin which is not well absorbable through the digestive tracts of animal and/or is likely to be enzymatically degraded into inactive components in vivo within the animal body is orally administered into the animal, the urinary recovery of the original, active cephalosporin which can be excreted into the urine without being degraded is very much low or null. Accordingly, when a test cephalosporin is orally administered, it is possible to estimate the degree of absorbability of the test cephalosporin by the digestive tracts and/or the degree of tolerability of the test cephalosporin against the "in vivo" enzymes, by evaluating the amount of the tested active cephalosporin which was excreted into and remaining in the urine, and hence the urinary recovery of the cephalosporin upon its oral administration. Thus, an evaluated higher urinary recovery of a test cephalosporin in the urine indicates that the test cephalosporin as orally administered has been absorbed better through the digestive tracts and then excreted into the urine with involving no or little degradation thereof.

The following test was hence made to measure the urinary recovery of the new cephalosporins of this invention.

Test 1

To mice (ICR-strain, female, 4 weeks-aged) was orally administered a new cephalosporin compound according to this invention at a dosage of 300 mcg per mouse. Until the end of 5 hours after oral administration of the test cephalosporin compound, all the volumes of urine discharged by the treated mice were collected together, and the total amount of the test compound remaining in the collected urine was measured by a microbial assay method.

The urinary recovery of the test compound was calculated in terms of percentage of the molar quantity of the test compound as recovered, against the molar quantity of the test compound as orally given. The test results are summarized in Table 2 below.

Table 2

| Test compound | Urinary recovery (%) |
|---|---|
| Example 1 Compound | 65.1 |
| Example 2 Compound | 48.3 |
| Example 3 Compound | 65.0 |
| Example 4 Compound | 58.8 |
| Example 5 Compound | 47.9 |
| Example 6 Compound | 32.9 |

From the results of the above Table 2, it will be apparent that the cephalosporin compound of the general formula (I) according to this invention when orally administered is easily absorbed through the digestive tracts of a living animal and maintains its antibacterial activity to a substantial extent in the body of the animal until it is excreted in the urine of the animal.

When the cephalosporin compound of the general formula (I) according to this invention is administered for therapeutic purpose, the compound (I) may be formulated into a conventional preparation form suitable for oral administration such as capsules, tablets or dry syrup according to a method usually practiced in the art, with optional addition of various additives such as excipient, binder and lubricant.

Description will next be made of the production of the new compounds of this invention.

The cephalosporin compounds of the general formula (I) may be prepared by condensing a 7-amino-3-cephem derivative having the general formula (A)

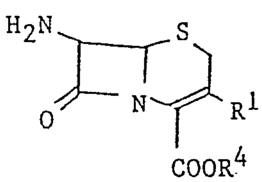

(A)

wherein $R^1$ is as defined above and $R^4$ is a mono-valent carboxyl-protecting group, for example, an aralkyl group such as diphenylmethyl and 4-methoxybenzyl, with a protected D-forphenicinol derivative having the general formula (B)

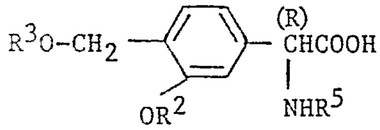

(B)

wherein $R^2$ and $R^3$ are as defined above and $R^5$ is a mono-valent amino-protecting group, for example, an alkoxycarbonyl group such as tertiary-butoxycarbonyl, or an aralkyloxycarbonyl such as p-methoxybenzyloxycarbonyl, in a conventional manner known per se for the synthesis of peptides, followed by removal from the condensation product of the residual protecting groups, namely, the amino-protecting group ($R^5$) such as tertiary butoxycarbony group) and the carboxyl-protecting group ($R^4$) (such as diphenylmethyl group and 4-methoxybenzyl group) by conventional deprotecting techniques.

In a second aspect of this invention, therefore, there is provided a process for the production of a cephalosporin compound having the general formula (I)

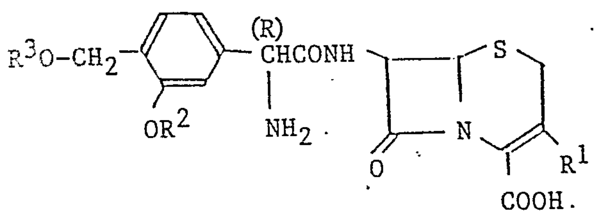

(I)

wherein $R^1$ is a lower alkyl group, a lower alkoxymethyl group or a lower alkenyl group, and $R^2$ and $R^3$ are each a hydrogen atom or a lower alkyl group, which comprises condensing a 7-amino-3-cephem derivative having the formula (A)

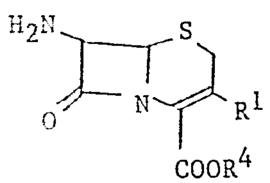

(A)

wherein $R^1$ is as defined above and $R^4$ is a mono-valent carboxyl-protecting group, with a D-forphenicinol derivative having the formula (B)

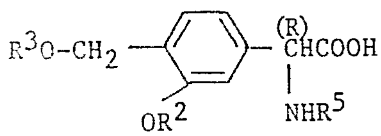

(B)

wherein $R^2$ and $R^3$ are as defined above and $R^5$ is an amino-protecting group, in an organic solvent to form a condensation product having the formula (Ia)

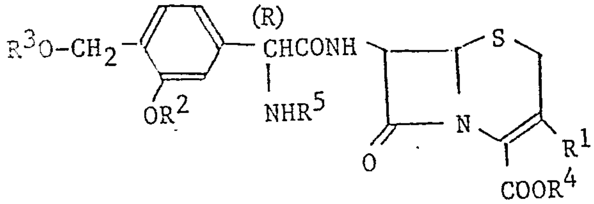

(Ia)

wherein $R^1$, $R^2$, $R^3$, $R^4$ and $R^5$ are as defined above, and then removing the remaining amino-protecting group

($R^5$) and the remaining carboxyl-protecting group ($R^4$) from the condensation product of the formula (Ia) to give the compound of the formula (I).

In the process of the first aspect of this invention, the condensation reaction between the compound of the formula (A) and the compound of the formula (B) may be carried out in an organic solvent at a temperature of 0°C to the refluxing temperature of the solvent employed, and preferably at a temperature of 20 - 30°C. The organic solvent used here may be selected from N,N-dimethylformamide, dioxane, acetonitrile, chloroform, methylene chloride, ethylene chloride, tetrahydrofuran, ethyl acetate and pyridine.

The compound of the formula (A) and the compound of the formula (B) may be reacted with each other in equimolar proportions or in a slight excess of either one of these compounds (A) and (B). The condensation reaction between the compound of the formula (A) and the compound of the formula (B) may preferably be carried out by known methods for amido synthesis such as methods using carbodiimide, mixed anhydride, azide, acid halide, active ester, etc. For instance, the active ester method using N-hydroxybenzotriazole or N-hydroxysuccinimide and N,N′-dicyclohexylcarbodiimide is preferred.

In the present process, the removal of the remaining amino-protecting group ($R^5$) and the remaining carboxyl-protecting group ($R^4$) from the condensation product of the formula (Ia) may be successively effected by the conventional deprotecting methods known per se in the art, which may suitably be chosen in accordance with the kinds of the protecting groups to be removed. For the removal of the amino-protecting group ($R^5$), any conventional deprotecting method such as hydrolysis or reduction may be effected. Acidic hydrolysis may conveniently be effected for the removal of an amino-protecting group, such as an alkoxycarbonyl, formyl or trityl group. Exemplary acid useful for this hydrolysis includes an organic and inorganic acid such as formic acid, trifluoroacetic acid, p-toluenesulfonic acid or hydrochloric acid, preferably, formic acid, trifluoroacetic acid, hydrochloric acid and the like. The hydrolyzing reaction to this end may be conducted either in the absence of any solvent or in the presence of water, a water-miscible organic solvent or a mixed solvent thereof. The acidic hydrolysis with trifluoroacetic acid may be carried out in the presence of anisole. Such an amino-protecting group of the aralkyloxycarbonyl type, for example, benzyloxycarbonyl group may be removed by catalytic hydrogenolysis in the presence of a palladium catalyst in a known manner. For the removal of the carboxyl-protecting group ($R^4$), a conventional deprotecting method such as hydrolysis or reduction can again be effected. The hydrolysis with acid may conveniently be effected for the removal of a carboxyl-protecting group ($R^4$) such as an aralkyl group or diphenylmethyl group.

For instance, according to the process of the second aspect of this invention, such compound of the formula (I), where $R^1$ is methyl group, may be produced by condensation of the compound of the formula (B) with such a starting compound of the formula (A), where $R^1$ is methyl group and $R^4$ is a carboxyl-protecting group, e.g. an aralkyl group, which has been prepared by esterifying the free 4-carboxyl group of such a compound of the formula (A), where $R^1$ is methyl group and $R^4$ is a hydrogen atom, as an initial material, with the carboxyl-protecting group, e.g. the aralkyl group.

Furthermore, such compound of the formula (I), where $R^1$ is an alkoxymethyl group, may be produced with starting from such compound of the formula (A), where $R^1$ is the alkoxymethyl group and $R^4$ is p-methoxybenzyl group as the carboxyl-protecting group, which may, in turn, be prepared from a known compound, 4-methoxybenzyl 3-chloromethyl-7-phenylacetamido-3-cephem-4-carboxylate of the formula (C)

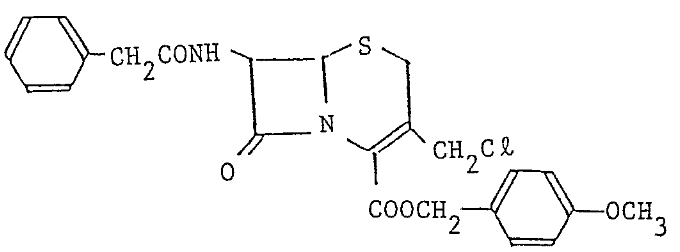

(C) .

as the initial material. Thus, in this case, the compound of the formula (C) is reacted with sodium iodide in an organic solvent such as acetone, to obtain 4-methoxybenzyl 3-iodomethyl-7-phenylacetamido-3-cephem-4-carboxylate, and then this iodination product is reacted with a lower alkanol in the presence of a catalyst (e.g., mercuric nitrate) to effect the reaction of said alkanol with the 3-iodomethyl group of said iodination product for the alkoxylation, thereby producing 4-methoxybenzyl 3-alkoxymethyl-7-phenylacetamido-3-cephem- 4-carboxylate (see Example 2(a) shown hereinafter). This 3-alkoxymethyl-3-cephem derivative so produced may then be treated in a conventional manner for the removal of the N-phenylacetyl group at the 7-position of said cephem derivative, for example, by reacting with phosphorus pentachloride in pyridine in an organic solvent such as dichloromethane and subsequently reacting with methanol (see Example 2(b) shown hereinafter). In this way, said compound of the formula (A), where $R^1$ is the alkoxymethyl group and $R^4$ is the p-methoxybenzyl group may be prepared. This compound of the formula (A) containing the alkoxymethyl group for the group $R^1$ is then condensed with the compound of the formula (B) in accordance with the process of the second aspect of this invention.

Moreover, such compound of the formula (I), where $R^1$ is an alkenyl group, may be produced also with using the compound of the formula (C) as the initial material. Thus, the compound of the formula (C) is reacted with sodium iodide and triphenylphosphine in an organic solvent such as dimethylformamide (DMF) to produce 4-(4-methoxybenzyloxycarbonyl)-7-phenylacetamido-3-cephem-3-yl-methyltriphenylphosphonium iodide, which is then reacted with a lower alkyl aldehyde (including formaldehyde) in the presence of a basic compound such as sodium carbonate according to the Wittig reaction to obtain a 4-methoxybenzyl 7-phenylacetamido-3-alkenyl-3-cephem-4-carboxylate (see Example 3(a) shown hereinafter). The latter carboxylate may then be treated in the above-mentioned conventional manner for the removal of the 7-N-phenylacetyl group therefrom (see Example 3(b) shown hereinafter), to afford the corresponding 4-methoxybenzyl 3-alkenyl-3-cephem-4-carboxylate, namely the compound of the formula (A) where $R^1$ is the lower alkenyl group and $R^4$ is the p-methoxybenzyl group. The resulting compound of the formula (A) containing a lower alkenyl group for the group $R^1$ may then be condensed with the compound of the formula (B) in accordance with the process of the second aspect of this invention, to afford the aimed compound of the formula (I) where $R^1$ is the alkenyl group.

On the other hand, according to this invention, such compound of the formula (I), where the group $R^2$ or both the groups $R^2$ and $R^3$ is or are a lower alkyl group, may be produced via a different route, by condensing or coupling a compound of the formula (A) where $R^1$ and $R^4$ are each as defined above, with such a compound of the formula (B) where $R^2$ and $R^3$ are each a hydrogen atom and $R^5$ is as defined above, to produce a condensation or coupled product having the formula (I) but where $R^2$ and $R^3$ are each the hydrogen atom and $R^1$, $R^4$ and $R^5$ are as defined above, and then reacting the resulting condensation or coupled product with a lower diazoalkane, preferably in the presence of a Lewis acid, so as to effect the O-alkylation of the 3-hydroxyl substituent and optionally also the 4-hydroxylmethyl substituent on the phenyl group of said condensation product, and finally removing the amino-protecting group ($R^5$) and the carboxyl-protecting group ($R^4$) from the alkylation product.

Thus, in a third aspect of this invention, there is provided a process for the production of a cephalospoprin compound having the general formula (I′)

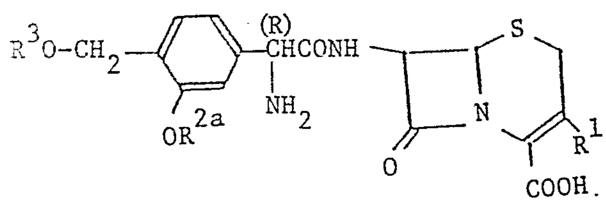

(I')

wherein $R^1$ is a lower alkyl group, a lower alkoxymethyl group or a lower alkenyl group, and $R^{2a}$ is a lower alkyl group and $R^{3a}$ is a hydrogen atom or a lower alkyl group, which comprises(i) alkylating the 3-hydroxyl substituent on the phenyl group of a protected cephalosporin derivative having the formula (Ib)

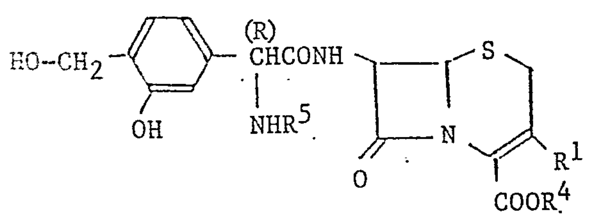

(Ib)

wherein $R^1$ is as defined above, $R^4$ is a carboxyl-protecting group and $R^5$ is an amino-protecting group, by reacting it with a lower diazoalkane or a trimethylsilyldiazo-(lower)alkane in an organic solvent to form a mono-O-alkylated product of the formula (Ic)

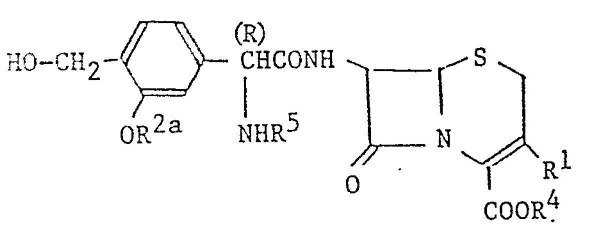

(Ic)

wherein $R^1$, $R^4$, $R^5$ and $R^{2a}$ are as defined above, and then, if desired, (ii) alkylating the 4-hydroxylmethyl substituent on the phenyl group of the mono-O-alkylated product of the formula (Ic) by reacting it with a lower diazoalkane or a trimethylsilyldiazo-(lower)alkane in an organic solvent, to produce the di-O-alkylated product of the formula (Id)

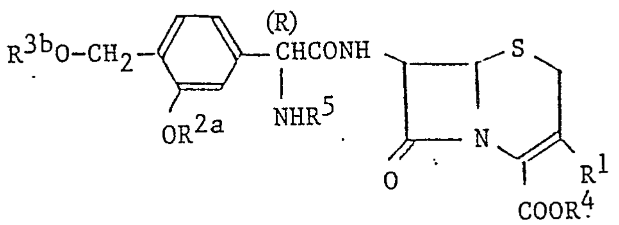

(Id)

wherein $R^1$, $R^4$, $R^5$ and $R^{2a}$ are asdefined above and $R^{3b}$ is the lower alkyl group, and (iii) removing the remaining amino-protecting group ($R^5$) and the remaining carboxyl-protecting group ($R^4$) from the mono-O-alkylated product of the formula (Ic) or the di-O-alkylated product of the formula (Id) to produce the cephalosporin compound of the formula (I').

In the process of the third aspect of this invention, the preparation of the starting compound of the formula (Ib) may be achieved by condensing a suitable compound of the formula (A) with a suitable compound of the formula (B) in the same manner as in the process of the second aspect of this invention.

In the present process, the compound of the formula (Ib) or the compound of the formula (Ic) may be reacted with an equimolar or substantially equimolar proportion of the lower diazoalkane or trimethylsilyldiazo(lower)alkane in an organic solvent under anhydrous conditions and the reaction may prefrably be effected at a temperature of 0°C to the refluxing temperature of the organic solvent employed, especially at room temperature, in the presence of a Lewis acid.

The lower diazoalkane to be reacted with the starting cephalosporin compound of the formula (Ib) may be represented by the formula $R^{2a}$-$N_2$ where $R^{2a}$ is the lower alkyl group, and the lower diazoalkane to be reacted with the intermediate mono-O-alkylated product of the formula (Ic) may also be represented by the formula $R^{3b}$-$N_2$ where $R^{3b}$ is the lower alkyl group. These lower diazoalkane may equally be diazomethane, diazoethane, diazopropane and the like. In stead of the diazoalkane, there may be used likewise its trimethylsilyl derivative having the formula $(CH_3)_3$-Si-$R^6$-$N_2$ where $R^6$ is a lower alkylene group. The reaction of the lower diazoalkane or trimethylsilyldiazo-lower alkane with the compound of the formula (Ib) or (Ic) may be effected in an inert organic solvent such as halogenated hydrocarbons, e.g. dichloroethane, chloroform, ethyl acetate and the like. The Lewis acid available in this O-alkylation includes boron trifluoride etherate and aluminium trichloride, and it is used in a catalytic quantity.

According to a further aspect of this invention, there is provided a pharmaceutical, antibacterial composition which comprises an antibacterially effective amount of the compound of the formula (I) or a pharmaceutically acceptable salt thereof as the active ingredient, in combination of a pharmaceutically acceptable carrier for the active ingredient.

The pharmaceutically acceptable carrier as mixed with the active ingredient compound may be an ordinary solid or liquid one, either organic or inorganic, which may be chosen appropriately depending on whether the pharmaceutical formulation as prepared is to be administered orally or non-orally or applied externally. The pharmaceutical composition of this invention may be of any conventional formulation form such as capsules, tablets, sugar-coated pills, ointment, suppository, solution, suspension and emulsion. Other conventional additives, including adjuvant, stabilizing agent, wetting agent, emulsifying agent, buffer solution may also be incorporated into the pharmaceutical composition of this invention containing the compound of the formula (I) as the active ingredient.

This invention is now illustrated with reference to the following Examples to which this invention is not limited.

Example 1

Production of 7-{(R)-2-amino-2-(3-hydroxy-4-hydroxymethylphenyl)acetamido}-3-methyl-3-cephem-4-carboxylic acid

(a) In N,N-dimethylformamide (hereinafter abbreviated as DMF) (1.5 ml) were dissolved N-(t-butoxycarbonyl)-D-forphenicinol (hereinafter abbreviated as Boc-D-For) (60 mg), 4-diphenylmethyl 7-amino-3-methyl-3-cephem-4-carboxylate (76 mg) and N-hydroxybenzotriazole (hereinafter abbreviated as HOBT; an active ester-forming reagent)(30 mg), and the resulting solution was cooled to 5°C. To the cooled solution was added N,N'-dicyclohexylcarbodiimide (hereinafter abbreviated as DDC; a condensing agent) (45.5 mg), and the mixture was kept at 25°C for 2 hours to effect the condensation reaction. The reaction solution was concentrated under reduced pressure to remove the solvent therefrom. The residue obtained was taken up into ethyl acetate (20 ml), and the resulting solution was filtered to remove the remaining insoluble matters. The filtrate was washed with water and then with saturated aqueous sodium chloride, and the washed filtrate

was dried over anhydrous sodium sulfate. The dried solution was evaporated under reduced pressure to remove the solvent (ethyl acetate) and obtain a residue. This residue was added with hexane to deposit a solid precipitate. For purification, the precipitate was dissolved in chloroform, and subjected to a column chromatography on a column of silica gel (as developed with chloroform-methanol (97:3) as eluent), whereby 81 mg of 4-diphenylmethyl 7-{(R)-2-(t-butoxycarbonylamino)-2-(3-hydroxy-4-hydroxymethylphenyl) acetamido}-3-methyl-3-cephem-4-carboxylate was obtained as the condensation product.
Melting point : about 125°C (with decomposition)
SIMS : 660 ($MH^+$)

(b) Trifluoroacetic acid (1 ml) was added dropwise to the 4-diphenylmethyl 7-{(R)-2-(t-butoxycarbonylamino)2-(3-hydroxy-4-hydroxymethylphenyl)acetamido}-3-methyl-3-cephem-4-carboxylate (70 mg) at 5°C in the presence of anisole (0.1 ml), and the resulting mixture was stirred for one hour, whereby the amino-protecting t-butoxycarbonyl group and the carboxyl-protecting 4-diphenylmethyl group were removed from the condensation product. Isopropyl ether was added to the reaction solution, which was then concentrated under reduced pressure. The residue obtained was further mixed with isopropyl ether, and a solid product precipitated was collected by filtration. The collected solid product was washed with isopropyl ether and then with diethyl ether. The powdery substance obtained was dissolved in water to a concentration of 10 mg per ml of water, and the aqueous solution was filtered to remove the insoluble matters therefrom. The filtrate was adjusted to pH 5 to 5.6 by addition of an ion-exchange resin, "Amberlite" IR-45(OH-form). The ion-exchange resin was removed by filtration and the filtrate was lyophilized to give 7-{(R)-2-amino-2-(3-hydroxy-4-hydroxymethylphenyl)acetamido}-3-methyl-3-cephem-4-carboxylic acid (29.5 mg).
Melting point : higher than 180°C (with decomposition)
SIMS : 394 ($MH^+$)

Example 2

Production of 7-{(R)-2-amino-2-(3-hydroxy-4-hydroxymethylphenyl)acetamido}-3-methoxymethyl-3-cephem-4-carboxylic acid

(a) 4-Methoxybenzyl 7-phenylacetamido-3-chloromethyl-3-cephem-4-carboxylate (486 mg) was dissolved in acetone (10 ml), to which was then added sodium iodide (375 mg). The resultant mixture was stirred for 2 hours at ambient temperature for the reaction. The reaction solution was concentrated under reduced pressure to remove the solvent, and the residue was dissolved in ethyl acetate. The solution was washed successively with water, 10% aqueous sodium thiosulfate and saturated aqueous sodium chloride, dried over anhydrous sodium sulfate and then concentrated under reduced pressure, whereby the iodination derivative, namely 4-methoxybenzyl 7-phenylacetamido-3-iodomethyl-3-cephem-4-carboxylate was obtained.

This iodination derivative was dissolved in methanol (2 ml), to which was then added a suspension of mercuric nitrate (343 mg) in acetonitrile (14 ml). The resultant mixture was then stirred for 20 minutes at ambient temperature for the reaction. The reaction solution was filtered to remove the insoluble matters therefrom, and the filtrate was mixed with water and extracted with ethyl acetate.

The organic phase, namely the extract in ethyl acetate was washed with water, dried over anhydrous sodium sulfate, and then concentrated under reduced pressure.

For the purification, the resulting residue was subjected to a column chromatography on silica gel (as developed with chloroform-methanol (49:1) as eluent). 4-Methoxybenzyl 7-phenylacetamido-3-methoxymethyl-3-cephem-4-carboxylate (240 mg) was thus obtained.

(b) Phosphorous pentachloride (483 mg) was suspended in dichloromethane (6 ml), to which was then added pyridine (184 mg) at 5°C, followed by stirring the resultant mixture at the same temperature for one hour. To the mixture was further added the 4-methoxybenzyl 7-phenylacetamido-3-methoxymethyl-3-cephem-4-carboxylate (373 mg), and the mixture obtained was stirred for 1.5 hours at 8 to 10°C as a preliminary stage for the reaction for the removal of the phenylacetyl group. The reaction solution was cooled to -35°C and then added with methanol (3.1 ml), and the mixture was stirred for 1.5 hours at -15°C. The reaction solution obtained was mixed with ice-cooled saturated aqueous sodium chloride (12 ml) and dichloromethane (12 ml). The resulting mixture was allowed to separate into the aqueous phase and the organic phase. The aqueous phase was separated from the organic phase and then extracted with dichloromethane. The organic extract in dichloromethane was combined with said organic phase, and the combined organic solution was washed with aqueous sodium hydrogen carbonate and saturated aqueous sodium chloride successively under ice-cooling. The washed organic solution was dried over anhydrous sodium sulfate and concentrated under reduced pressure to give 4-methoxybenzyl 7-amino-3-methoxymethyl-3-cephem-4-carboxylate (250 mg).

(c) Boc-D-For (200 mg), the 4-methoxybenzyl 7-amino-3-methoxymethyl-3-cephem-4-carboxylate (245 mg) and HOBT (100 mg) were dissolved in DMF (4 ml) and the solution was cooled to 5°C. To the cooled solution was added DCC (153 mg), and the resultant mixture was stirred for 2 hours at 25°C, to effect the condensation reaction between the Boc-D-For and the 3-cephem-4-carboxylate compound. The resulting reaction solution was concentrated under reduced pressure and the residue was taken up into ethyl acetate (50 ml). The resulting solution was filtered to remove the insoluble matters therefrom, and the filtrate was washed with water and saturated aqueous sodium chloride successively, and dired over anhydrous sodium sulfate. The dried solution was distilled under reduced pressure to remove the solvent, and the syrupy residue was

admixed with hexane to deposit a precipitate. For the purification, the precipitate was subjected to a columm chromatography on silica gel (as developed with chloroform-methanol (97:3) as eluent). 4-Methoxybenzyl 7-{(R)-2-(t-butoxycarbonylamino)2-(3-hydroxy-4-hydroxymethylphenyl)acetamido}-3-methoxymethyl-3-cephem-4-carboxylate (312 mg) was thus obtained.
Melting point : 110 - 118°C (with decomposition)
SIMS : 643 ($MH^+$)

(d) To the 4-methoxybenzyl 7- {(R)-2-(t-butoxycarbonylamino)-2-(3-hydroxy-4-hydroxymethylphenyl) acetamido}-3-methoxymethyl-3-cephem-4-carboxylate (100 mg) was added dropwise trifluoroacetic acid (1 ml) in the presence of anisole (0.1 ml) at 5° C, and the mixture was stirred for one hour to effect the reaction for the removal of the t-butoxycarbonyl and 4-methoxybenzyl groups. The reaction solution was admixed with isopropyl ether and concentrated under reduced pressure, and the concentrated solution was further added with isopropyl ether to deposit a precipitate. The precipitate was collected by filtration and washed with isopropyl ether and diethyl ether successively. The powdery product obtained was dissolved in water to the concentration of 10 mg per ml of water, and the aqueous solution was filtered to remove the insoluble matters. The filtrate was adjusted to pH 5 to 5.6 by addition of an ion-exchange resin, "Amberlite" IR-45 (OH-form). The ion-exchange resin was removed by filtration and the filtrate was lyophilized to give 7- {(R)-2-amino-2-(3-hydroxy-4-hydroxymethylphenyl)acetamido}-3-methoxymethyl-3-cephem-4-carboxylic acid (52 mg) as the desired compound.
Melting point : above 172° C (with decomposition)
SIMS : 424 ($MH^+$)

Example 3

Production of 7-{(R)-2-amino-2-(3-hydroxy-4-hydroxymethylphenyl)acetamido}-3-vinyl-3-cephem-4-carboxylic acid

(a) 4-Methoxybenzyl 7-phenylacetamido-3-chloromethyl-3-cephem-4-carboxylate (2.43 g) was dissolved in DMF (7 ml), to which were then added sodium iodide (900 mg) and triphenylphosphine (1.44 g) under ice-cooling. The resultant mixture was stirred for 2 hours at ambient temperature to effect the reaction. The reaction solution was added to isopropyl alcohol (200 ml) to deposit a precipitate, which was collected by filtration. This precipitate comprised 4-(4-methoxybenzyloxycarbonyl)-7-phenylacetamido-3-cephem-3-yl-methyltriphenylphosphonium iodide. This substance was dissolved in a mixture of dichloromethane (7 ml) and water (3.5 ml). The solution was mixed with 36% aqueous formaldehyde (11.5 ml) and then adjusted to pH 9.0 with 20% aqueous sodium carbonate. The resulting mixture was stirred at ambient temperature for one hour, during which the Wittig reaction took place between the formaldehyde and the phosphonium iodide compound to form 4-methoxybenzyl 7-phenylacetamido-3-vinyl-3-cephem-4-carboxylate. The reaction solution was adjusted to pH 5 with 10% aqueous hydrochloric acid and extracted with dichloromethane. The organic phase, namely the extract in dichloromethane was washed with water, dried over anhydrous sodium sulfate, filtered and concentrated under reduced pressure. The residue obtained was washed with toluene, to afford the 4-methoxybenzyl 7-phenylacetamido-3-vinyl-3-cephem-4-carboxylate (1.8 g).

(b) Phosphorus pentachloride (1.25 g) was suspended in dichloromethane (15 ml), to which was then added pyridine (480 mg) at 5° C, followed by stirring the resulting mixture for one hour at the same temperature (at 5° C). The mixture was mixed with the 4-methoxybenzyl 7-phenylacetamido-3-vinyl-3-cephem-4-carboxylate (928 mg), followed by agitation for 1.5 hours at 8° to 10° C for the preliminary reaction to remove the phenylacetyl group. The reaction solution was cooled to -35° C and then added with methanol (8 ml), followed by stirring the resultant mixture for 1.5 hours at -15° C. This reaction solution obtained was further mixed with ice-cooled saturated aqueous sodium chloride (40 ml) and dichloromethane (40 ml). The resulting mixture was allowed to separate into the aqueous phase and the organic phase. The aqueous phase was separated from the organic phase and extracted with dichloromethane. The organic extract in dichloromethane was combined with said organic phase, and the combined organic solution was washed successively with aqueous sodium hydrogen carbonate and saturated aqueous sodium chloride under ice-cooling, dried over anhydrous sodium sulfate, and concentrated under reduced pressure to give 4-methoxybenzyl 7-amino-3-vinyl-3-cephem-4-carboxylate (750 mg).

(c) Boc-D-For (110 mg), the 4-methoxybenzyl 7-amino-3-vinyl-3-cephem-4-carboxylate (128 mg) and HOBT (55.5 mg) were dissolved in DMF (2 ml), and the solution was cooled to 5° C. The solution was added with DCC (84.5 mg), and the resultant mixture was stirred for 2 hours at 25° C to effect the condensation reaction. The reaction solution was concentrated to remove the solvent, and the residue was added with ethyl acetate (25 ml). The solution obtained was filtered to remove the insoluble matters, and the filtrate was washed successively with water and saturated aqueous sodium chloride. The washed solution (the filtrate) was dried over anhydrous sodium sulfate, concentrated under reduced pressure, and the syrupy residue was mixed with hexane to deposite a precipitate. For the purification, the precipitate was subjected to a column chromatography on silica gel (as developed with chloroform-methanol (97:3) as eluent). In this way, 4-methoxybenzyl 7- {(R)-2-(t-butoxycarbonylamino)2-(3-hydroxy-4-hydroxymethylphenyl)acetamido}-3-vinyl-3-cephem-4-carboxylate (158 mg) was obtained.
Melting point : about 112° C (with decomposition)
SIMS : 626 ($MH^+$)

(d) To the 4-methoxybenzyl 7-{(R)-2-(t-butoxycarbonylamino)-2-(3-hydroxy-4-hydroxymethylphenyl) acetamido}-3-vinyl-3-cephem-4-carboxylate (100 mg) was added dropwise trifluoroacetic acid (1 ml) in the presence of anisole (0.1 ml) at 5°C, and the resulting mixture was stirred for one hour to effect the hydrolysis reaction for the removal of the t-butoxycarbonyl and 4-methoxybenzyl groups. The reaction solution was mixed with isopropyl ether and concentrated under reduced pressure, followed by mixing the resultant concentrate with isopropyl ether to deposit a precipitate. The precipitate was collected by filtration and washed with isopropyl ether and then with diethyl ether. The powdery product obtained was dissolved in water to the concentration of 10 mg/ml and the solution was filtered to remove the insoluble matters. The resultant filtrate was adjusted to pH 5 to 5.6 by addition of an ion-exchange resin, "Amberlite" IR-45 (OH-form) and then filtered again to remove the resin. The filtrate was then lyophilized to give the desired compound, 7-{(R)-2-amino-2-(3-hydroxy-4-hydroxymethylphenyl)acetamido}-3-vinyl-3-cephem-4-carboxylic acid (47 mg).
Melting point : above 184°C (with decomposition)
SIMS : 406 ($MH^+$)

Example 4

Production of 7-{(R)-2-amino-2-(3-hydroxy-4-hydroxymethylphenyl)acetamido}-3-{(Z)-1-propenyl}-3-cephem-4-carboxylic acid

(a) 4-(4-Methoxybenzyloxycarbonyl)-7-phenylacetamido-3-cephem-3-yl-methyltriphenylphosphonium iodide (1.6 g) as obtained in the process of the Example 3(a) above was dissolved in a mixture of dichloromethane (5 ml) and water (6 ml), to which was then added 90% aqueous acetaldehyde (3 ml). The resultant mixture was adjusted to pH 9.0 with 20% aqueous sodium carbonate and then was allowed to undergo the Wittig reaction for one hour. The reaction solution was adjusted to pH 5.0 with 10% aqueous hydrochloric acid and extracted with dichloromethane. The organic layer, namely the extract solution in dichloromethane was washed with water, dried over anhydrous sodium sulfate and concentrated under reduced pressure. For the purification, the resultant residue was subjected to a column chromatography on silica gel (as developed with chloroform-methanol (49:1) as eluent). In this way, 4-methoxybenzyl 7-phenylacetamido-3-{(Z)-1-propenyl}-3-cephem-4-carboxylate (482 mg) was afforded.

(b) Phosphorus pentachloride (483 mg) was suspended in dichloromethane (6 ml), to which was then added pyridine (184 mg) at 5°C, and the resultant mixture was stirred for one hour at the same temperature. To the mixture was further added the 4-methoxybenzyl 7-phenylacetamido-3-{(Z)-1-propenyl}-3-cephem-4-carboxylate (370 mg), followed by stirring for 1.5 hours at 8 to 10°C to effect the reaction for the removal of the phenylacetyl group. The reaction solution was cooled to -35°C, mixed with methanol (3.1 ml) and stirred for 1.5 hours at -15°C. The reaction solution so obtained was then mixed with ice-cooled saturated aqueous sodium chloride (12 ml) and dichloromethane (12 ml). The mixture was allowed to separate into the aqueous phase and the organic phase. The aqueous phase was separated from the organic phase and was extracted with dichloromethane. The organic extract obtained was combined with said organic phase and the combined solution was washed successively with aqueous sodium hydrogen carbonate and saturated aqueous sodium chloride under ice-cooling. The washed solution was dried over anhydrous sodium sulfate and concentrated under reduced pressure to give 4-methoxybenzyl 7-amino-3-{(Z)-1-propenyl}-3-cephem-4-carboxylate (275 mg).

(c) Boc-D-For (223 mg), the 4-methoxybenzyl 7-amino-3-{(Z)-1-propenyl}-3-cephem-4-carboxylate (270 mg) and HOBT (112 mg) were dissolved in DMF (4 ml) and the solution was cooled to 5°C. To the solution was added DCC (170 mg), and the resultant mixture was stirred for 2 hours at 25°C to effect the condensation reaction. The reaction solution was concentrated under reduced pressure to remove the solvent therefrom and the residue was dissolved in ethyl acetate (50 ml). The resulting solution was filtered and the filtrate was washed successively with water and saturated aqueous sodium chloride, dried over anhydrous sodium sulfate and concentrated under reduced pressure. The concentrated solution was mixed with hexane to deposit a precipitate. For the purification, the aforesaid precipitate was subjected to a column chromatography on silica gel (as developed with chloroform-methanol (97 : 3) as eluent). In this way, 4-methoxybenzyl 7-{(R)-2-(t-butoxycarbonylamino)-2-(3-hydroxy-4-hydroxymethylphenyl)acetamido}-3-{(Z)-1-propenyl}-3-cephem-4-carboxylate (325 mg) was obtained.
Melting point : 104 - 112°C (with decomposition)
SIMS : 640 ($MH^+$)

(d) To the 4-methoxybenzyl 7-{(R)-2-(t- butoxycarbonylamino)-2-(3-hydroxy-4-hydroxymethylphenyl) acetamido}-3- {(Z)-1-propenyl}-3-cephem-4-carboxylate (100 mg) was added dropwise trifluoroacetic acid (1 ml) in the presence of anisole (0.1 ml) at 5°C, and the resulting mixture was stirred for one hour to effect the hydrolysis reaction for removal of the t-butoxycarbonyl and 4-methoxybenzyl groups. The reaction solution was mixed with isopropyl ether and concentrated under reduced pressure, followed by mixing the resultant concentrated solution with isopropyl ether to deposit a precipitate. The precipitate was collected by filtration and washed with isopropyl ether and then with diethyl ether. The powdery product so obtained was dissolved in water to the concentration of 10 mg/ml and the solution was filtered to remove the insoluble matters. The filtrate was adjusted to pH 5 - 5.6 by addition of an ion-exchange resin, "Amberlite" IR-45 (OH-form) and again filtered to remove the resin. The filtrate was then lyophilized to give the desired compound, 7-{(R)-2-amino--

2-(3-hydroxy-4-hydroxymethylphenyl)acetamido}-3- {(Z)-1-propenyl}-3-cephem-4-carboxylic acid (47 mg).
Melting point : above 175°C (with decomposition)
SIMS : 420 ($MH^+$)

Example 5

Production of 7- {(R)-amino-(4-hydroxymethyl-3-methoxyphenyl)acetamido}-3-{(Z)-1-propenyl}-3-cephem-4-carboxylic acid

(a) 4-Methoxybenzyl 7-{(R)-2-(t-butoxycarbonylamino)-2-(3-hydroxy-4-hydroxymethylphenyl)acetamido}-3-{(Z)-1-propenyl}-3-cephem-4-carboxylate (40 mg) which was obtained in the process of the Example 4(c) above was dissolved in chloroform (1 ml), to which was then added a solution (1.5 ml) of 10% trimethylsilyldiazomethane in hexane. The resultant mixture was allowed to undergo the reaction for 2.5 hours. The reaction solution was then concentrated under reduced pressure to remove the solvent therefrom, and the residue was purified by dissolving it in chloroform and subjecting the solution to a silica gel thin layer chromatography (as developed with chloroform-methanol (95 : 5) as eluent). Thus, there was afforded the mono-O-methylated derivative, namely 4-methoxybenzyl 7- {(R)-2-(t-butoxycarbonylamino)-2-(3-methoxy-4-hydroxymethylphenyl)acetamido}-3-{(Z)-1-propenyl}-3-cephem-4-carboxylate (15 mg).
Melting point : 85 - 88°C

(b) To said mono-O-methylated derivative (13 mg) was added dropwise trifluoroacetic acid (0.15 ml) in the presence of anisole(0.015 ml) at 5°C, and the mixture was stirred for one hour for the reaction to remove the t-butoxycarbonyl and 4-methoxybenzyl groups. The reaction solution was mixed with isopropyl ether and concentrated under reduced pressure, followed by mixing the concentrated solution with isopropyl ether to deposit a precipitate. The precipitate was collected by filtration and washed with isopropyl ether and with diethyl ether. The powdery product thus obtained was dissolved in water to the concentration of 10 mg/ml and the solution was filtered to remove the insoluble matters. The filtrate was adjusted to pH 5 - 5.6 by addition of an ion-exchange resin, "Amberlite" IR-45 (OH-form) and again filtered to remove the resin. The filtrate was lyophilized to give 7-{(R)-2-amino-2-(4-hydroxymethyl-3-methoxyphenyl)acetamido}-3-{(Z)-1-propenyl}-3-cephem-4-carboxylic acid (5.8 mg).

Example 6

Production of 7-{(R)-2-amino-2-(3-methoxy-4-methoxymethylphenyl)acetamido}-3-{(Z)-1-propenyl}-3-cephem-4-carboxylic acid

(a) 4-Methoxybenzyl 7-{(R)-2-(t-butoxycarbonylamino)-2-(3-methoxy-4-hydroxymethylphenyl)acetamido}-3-{(Z)-1-propenyl}-3-cephem-4-carboxylate (38 mg) (as obtained in Example 5(a) above) was dissolved in dichloromethane (1 ml). To the solution were added a catalytic amount of boron trifluoride etherate (as a Lewis acid) and a solution (1 ml) of 10% trimethylsilydiazomethane in hexane. The resultant mixture was stirred for 10 hours to effect the methylation of the cephem compound. The reaction solution was concentrated under reduced pressure to remove the solvent, and the residue was purified by silica gel thin layer chromatography (as developed with chloroform-methanol (95 : 5) as eluent). There was thus isolated the di-O-methylated derivative, namely 4-methoxybenzyl 7-{(R)-2-(t-butoxycarbonylamino)-2-(3-methoxy-4-methoxymethylphenyl)acetamido}-3-{(Z)-1-propenyl}-3-cephem-4-carboxylate (16 mg).

(b) To said di-O-methylated derivative (15 mg) was added dropwise trifluoroacetic acid (0.18 ml) in the presence of anisole (0.02 ml) at 0°C, and the resultant mixture was stirred for one hour to effect the reaction for removal of the t-butoxycarbonyl and 4-methoxybenzyl groups. The reaction solution was mixed with isopropyl ether and concentrated under reduced pressure. The concentrate solution was further mixed with isopropyl ether to deposit a precipitate, which was then collected by filtration and washed with isopropyl ether and then with diethyl ether. The powdery product thus obtained was dissolved in water to the concentration of 10 mg/ml, and the solution was filtered to remove the insoluble matters. The filtrate was adjusted to pH 5 - 5.6 by addition of an ion-exchange resin, "Amberlite" IR-45 (OH-form) and filtered again to remove the resin. The filtrate was subsequently lyophilized to give the desired compound, 7-{(R)-2-amino-2-(3-methoxy-4-methoxymethylphenyl)acetamido}-3-{(Z)-1-propenyl}-3-cephem-4-carboxylic acid (8.0 mg).

## Claims

1. A cephalosporin compound represented by the general formula (I)

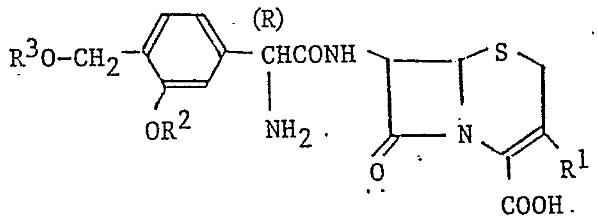

(I)

wherein $R^1$ is a lower alkyl group, a lower alkoxymethyl group or a lower alkenyl group, and $R^2$ and $R^3$ are each a hydrogen atom or a lower alkyl group, and a pharmaceutically acceptable salt thereof.

2. A cephalosporin compound as claimed in claim 1, wherein $R^1$ is a methyl group, and $R^2$ and $R^3$ are each a hydrogen atom, and a pharmaceutically acceptable salt thereof.

3. A cephalosporin compound as claimed in claim 1, wherein $R^1$ is a methoxymethyl group, and $R^2$ and $R^3$ are each a hydrogen atom, and a pharmaceutically acceptable salt thereof.

4. A cephalosporin compound as claimed in claim 1, wherein $R^1$ is a vinyl group, and $R^2$ and $R^3$ are each a hydrogen atom, and a pharmaceutically acceptable salt thereof.

5. A cephalosporin compound as claimed in claim 1, wherein $R^1$ is a 1-propenyl group, and $R^2$ and $R^3$ are each a hydrogen atom, and a pharmaceutically acceptable salt thereof.

6. A cephalosporin compound as claimed in claim 1, wherein $R^1$ is a 1-propenyl group, $R^2$ is a methyl group, and $R^3$ is a hydrogen atom, and a pharmaceutically acceptable salt thereof.

7. A cephalosporin compound as claimed in claim 1, wherein $R^1$ is a 1-propenyl group, and $R^2$ and $R^3$ are each a methyl group, and a pharmaceutically acceptable salt thereof.

8. A compound as claimed in claim 1 which is selected from 7-{(R)-2-amino-2-(3-hydroxy-4-hydroxymethylphenyl) acetamido}-3-methyl-3-cephem-4-carboxylic acid; and 7-{(R)-2-amino-2-(3-hydroxy-4-hydroxymethylphenyl) acetamido}-3-methoxymethyl-3-cephem-4-carboxylic acid and pharmaceutically acceptable salts thereof.

9. A compound as claimed in claim 1 which is selected from 7- {(R)-2-amino-2-(3-hydroxy-4-hydroxymethylphenyl) acetamido}-3-vinyl-3-cephem-4-carboxylic acid; 7-{(R)-2-amino-2-(3-hydroxy-4-hydroxymethylphenyl)acetamido}-3-{(Z)-1-propenyl}-3-cephem-4-carboxylic acid; 7-{(R)-2-amino-2-(4-hydroxymethyl-3-methoxyphenyl)acetamido}-3-{(Z)-1-propenyl}-3-cephem-4-carboxylic acid; and 7-{(R)-2-amino-2-(3-methoxy-4-methoxymethylphenyl)acetamido}-3-{(Z)-1-propenyl}-3-cephem-4-carboxylic acid, and pharmaceutically acceptable salts thereof.

10. A process for the production of a cephalosporin compound having the general formula (I)

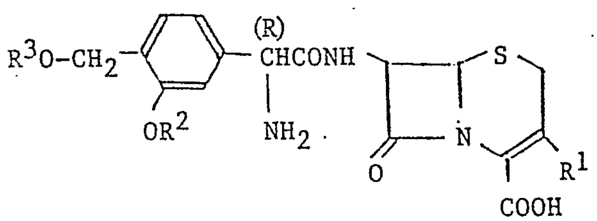

(I)

wherein $R^1$ is a lower alkyl group, a lower alkoxymethyl group or a lower alkenyl group, and $R^2$ and $R^3$ are each a hydrogen atom or a lower alkyl group, which comprises condensing a 7-amino-3-cephem derivative having the formula (A)

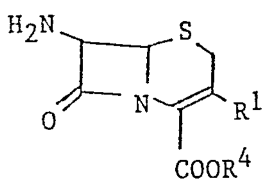

(A)

wherein $R^1$ is as defined above and $R^4$ is a mono-valent carboxyl-protecting group, with a D-forphenicinol derivative having the formula (B)

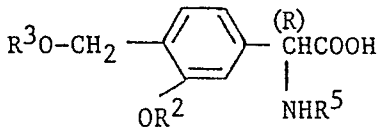

(B)

wherein $R^2$ and $R^3$ are as defined above and $R^5$ is an amino-protecting group, in an organic solvent to form a condensation product having the formula (Ia)

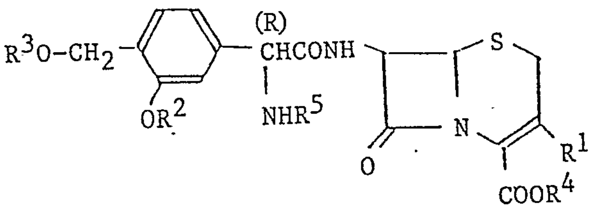

(Ia)

wherein $R^1$, $R^2$, $R^3$, $R^4$ and $R^5$ are as defined above, and then removing the remaining amino-protecting group ($R^5$) and the remaining carboxyl-protecting group ($R^4$) from the condensation product of the formula (Ia) to give the compound of the formula (I).

11. The process as claimed in claim 10 in which the condensation reaction between the compound of the formula (A) and the compound of the formula (B) is carried out in an organic solvent at a temperature of 0°C to the refluxing temperature of the solvent employed, and preferably at a temperature of 20 - 30°C, and the organic solvent is selected from N,N-dimethylformamide, dioxane, acetonitrile, chloroform, methylene chloride, ethylene chloride, tetrahydrofuran, ethyl acetate and pyridine.

12. The process as claimed in claim 10 in which the condensation reaction between the compound of the formula (A) and the compound of the formula (B) is carried out by active ester method using N-hydroxybenzotriazole and N,N′-dicyclohexylcarbodiimide.

13. A process for the production of a cephalosporin compound having the general formula (I′)

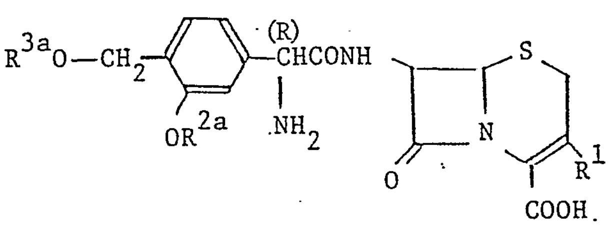

(I′)

wherein $R^1$ is a lower alkyl group, a lower alkoxymethyl group or a lower alkenyl group, and $R^{2a}$ is a lower alkyl group and $R^{3a}$ is a hydrogen atom or a lower alkyl group, which comprises (i) alkylating the 3-hydroxy substituent on the phenyl group of a protected cephalosprorin derivative having the formula (Ib)

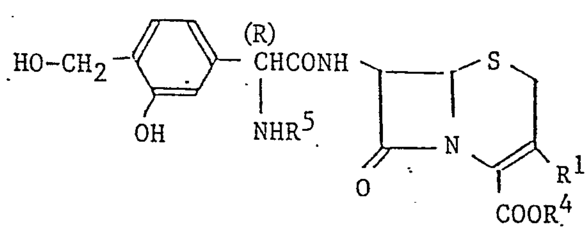

(Ib)

wherein $R^1$ is as defined above, $R^4$ is a carboxyl-protecting group and $R^5$ is an amino-protecting group, by reacting it with a lower diazoalkane or a trimethylsilyldiazo-(lower)alkane in an organic solvent to form a mono-O-alkylated product of the formula (Ic)

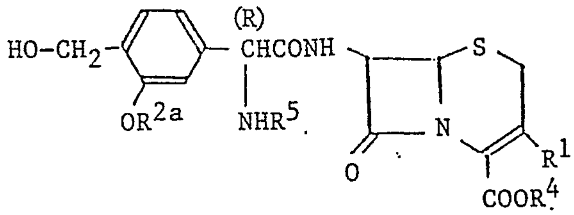

(Ic)

wherein $R^1$, $R^4$, $R^5$ and $R^{2a}$ are as defined above, and then, if desired, (ii) alkylating the 4-hydroxylmethyl substituent on the phenyl group of the mono-O-alkylated product of the formula (Ic) by reacting it with a lower diazoalkane or a trimethylsilyldiazo-(lower) alkane in an organic solvent, to produce the di-O-alkylated product of the formula (Id)

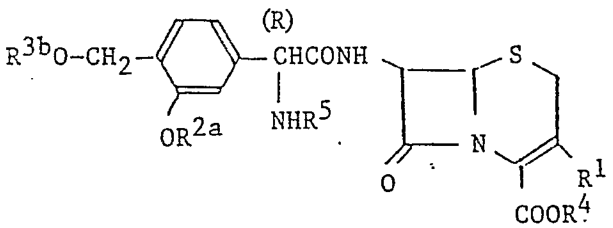

(Id)

wherein $R^1$, $R^4$, $R^5$ and $R^{2a}$ are as defined above and R3b is the lower alkyl group, and (iii) removing the remaining amino-protecting group ($R^5$) and the remaining carboxyl-protecting group ($R^4$) from the mono-O-alkylated product of the formula (Ic) or the di-O-alkylated product of the formula (Id) to produce the cephalosporin compound of the formula (I′).

14. The process as claimed in Claim 13, in which the compound of the formula (Ic) is reacted with an equimolar or substantially equimolar proportion of the lower diazoalkane or trimethylsilyldiazo-(lower)alkane in an organic solvent under anhydrous conditions at a temperature of 0°C to the refluxing temperature of the organic solvent employed, preferably at room temperature in the presence of a Lewis acid.

15. A pharmaceutical, antibacterial composition which comprises an antibacterially effective amount of the compound of the formula (I) as defined in Claim 1, as the active ingredient, in combination with a pharmaceutically acceptable carrier for the active ingredient.

16. Use of the cephalosporin compound of the formula (I) as claimed in claim 1 in a pharmaceutical composition.